# EUROPEAN PATENT APPLICATION

(11) **EP 4 279 601 A1**
(43) Date of publication of application: **22.11.2023**
(21) Application number: 22739004.4
(22) Date of filing: 11.01.2022
(51) Int. Cl.: C12P 13/24, C12N 1/21

(54) **PROTEIN HAVING L-PROLINE EFFLUX FUNCTION, AND USE THEREOF**

(30) Priority: 13.01.2021 CN 202110045175
(71) Applicant: Tianjin Institute of Industrial Biotechnology, Chinese Academy of Sciences, Tianjin Airport Economic Area Tianjin 300308 (CN)
(72) Inventor: SUN, Jibin, Tianjin 300308 (CN); LIU, Jiao, Tianjin 300308 (CN); ZHENG, Ping, Tianjin 300308 (CN); LIU, Moshi, Tianjin 300308 (CN); WANG, Yu, Tianjin 300308 (CN); ZHOU, Wenjuan, Tianjin 300308 (CN); SUN, Guannan, Tianjin 300308 (CN)
(74) Representative: Gille Hrabal Partnerschaftsgesellschaft mbB Patentanwälte
(86) International application number: PCT/CN2022/071346
(87) International publication number: WO 2022/152125

(57) **Abstract**

Disclosed in the present invention are a protein having an L-proline efflux function, and the use thereof. A method for producing L-proline or hydroxyproline by means of using a protein ThrE is used for producing L-proline by means of enhancing the activity of a polypeptide, having an L-proline efflux function, in an L-proline-producing strain. Alternatively, the method is used for producing hydroxyproline by means of weakening the activity of a polypeptide, having an L-proline efflux function, in L-proline-producing host cells and enhancing the activity of a proline hydroxylase.

## Description

The present application claims priority to Chinese Patent Application No. 2021100451754 filed with China National Intellectual Property Administration on Jan. 13, 2021, entitled "PROTEIN HAVING L-PROLINE EFFLUX FUNCTION, AND USE THEREOF", which is incorporated herein by reference in its entirety.

### TECHNICAL FIELD

The present disclosure belongs to the field of molecular biology and bioengineering, and particularly relates to novel use of ThrE, a threonine efflux protein of *Corynebacterium glutamicum,* as an L-proline efflux protein, and a method for producing L-proline and a derivative thereof by using the protein ThrE.

### BACKGROUND

L-proline is a naturally occurring non-essential amino acid in the human body and has a wide range of applications in the fields of clinics, biomaterials, industry, and the like. Methods for producing L-proline mainly include chemical methods and fermentation methods. The chemical extraction method has gradually lost its marketability due to the serious pollution and high cost, while the microbial fermentation method has become the most widely used method in the modern industry due to its advantages of low production cost, high productivity, high specificity, low environmental pollution, and the like. Currently, the commonly used industrial fermentation strains are Bacillus and Escherichia, commonly used Escherichia such as *Escherichia coli,* commonly used Bacillus such as *Corynebacterium glutamicum,* short bacillus such as *Brevibacterium flavum*, *Brevibacterium lactofermentus*, as well as some species of the genus Arthrobacter and some species of the genus Microbacterium. Among them, *Corynebacterium glutamicum* has physiological superiority and thus has become the most important strain used in industry for producing amino acids and other products.

In *Corynebacterium,* L-proline is produced mainly by glutamic acid as a substrate catalyzed by γ-glutamyl kinase (glutamate-5-kinase, ProB), glutamate-semialdehyde dehydrogenase (ProA), and pyrroline-5-carboxylate reductase (ProC). In the prior art, L-proline is mainly produced by genetic modifications of ProA and ProB, the key enzymes in a synthetic pathway of L-proline. For example, a mutation at position 149 in the ProB protein derived from *Corynebacterium glutamicum* can relieve the feedback inhibition of L-proline and increase the yield of L-proline in engineered strains, as reported in CN101084312A.

In the fermentation process, the timely transport of an amino acid synthesized in a microorganism to the outside of a cell through a transport pathway is also very important for promoting the biosynthesis of the amino acid. For example, in the production of lysine, the yield of lysine in an engineered strain can be remarkably increased by overexpression of a lysine efflux protein LysE (CN1283797C). However, the use of L-proline efflux protein to increase the yield of L-proline in the production of L-proline has not been reported. Therefore, there is an urgent need in the field to discover an effective L-proline efflux protein, so as to efficiently excrete L-proline produced in a cell to the outside of the cell and obtain a high-yield L-proline-producing engineered bacterium.

### SUMMARY

In order to overcome the problems in the prior art, in the present disclosure, L-proline efflux proteins of *Corynebacterium glutamicum* were screened by a whole genome-scale membrane transporter inhibition library. Based on the screening in the present disclosure, it was found that the inhibition of *thrE* (Cgl2622, which has been confirmed in the literature as the gene encoding the threonine efflux protein) gene resulted in a significant decrease in the specific productivity of L-proline. Overexpression of ThrE in a bacterium for producing L-proline, SZCgP1 (*Corynebacterium glutamicum* ATCC13869, in which a G149D mutation of ProB protein was introduced, with a codon mutation from GGT to GAT, see CN101084312A for details), resulting in a significantly higher extracellular proline concentration throughout the fermentation process and a significantly lower intracellular proline concentration throughout the fermentation process (especially during the rapid acidogenic phase), as compared to a control strain SZCgP1(pEC-control). The above results indicate that ThrE is an L-proline efflux protein.

An objective of the present disclosure is to provide the use of the proline efflux protein in producing L-proline or hydroxyproline.

Another objective of the present disclosure is to provide a strain producing L-proline or hydroxyproline.

Still, another objective of the present disclosure is to provide a method for producing L-proline or hydroxyproline.

Still, yet another objective of the present disclosure is to provide a method for constructing a strain producing L-proline or hydroxyproline.

In a first aspect of the present disclosure, provided is use of a polypeptide as a proline efflux protein in producing L-proline and hydroxyproline, the polypeptide being:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function.

In another preferred embodiment, the polypeptide is a polypeptide derived from a polypeptide having an amino acid sequence set forth in SEQ ID NO: 1 or 2, is formed by adding one or several, preferably 1-20, more preferably 1-15, more preferably 1-10, more preferably 1-3, and most preferably 1 amino acid residue at either or both ends of the amino acid sequence set forth in SEQ ID NO: 1 or 2, and has an L-proline efflux function.

In another preferred embodiment, the polypeptide has an amino acid sequence as set forth in SEQ ID NO: 1 or SEQ ID NO: 2.

In a second aspect of the present disclosure, provided is a strain for producing L-proline, wherein the strain expresses the following polypeptide:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function.

In another preferred embodiment, the polypeptide is a polypeptide derived from a polypeptide having an amino acid sequence set forth in SEQ ID NO: 1 or 2, is formed by adding one or several, preferably 1-20, more preferably 1-15, more preferably 1-10, more preferably 1-3, and most preferably 1 amino acid residue at either or both ends of the amino acid sequence set forth in SEQ ID NO: 1 or 2, and has an L-proline efflux function.

In another preferred embodiment, the polypeptide has an amino acid sequence as set forth in SEQ ID NO: 1 or SEQ ID NO: 2.

In another preferred embodiment, the strain is a bacterium.

In another preferred embodiment, the strain is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067.

In another preferred embodiment, glutamate kinase in the bacterium is not subjected to feedback inhibition by L-proline or is subjected to attenuated feedback inhibition by L-proline.

In another preferred embodiment, the activity of glutamate kinase and/or glutamate-semialdehyde dehydrogenase and/or pyrroline-5-carboxylate reductase in the bacterium is enhanced.

In a third aspect of the present disclosure, provided is a method for producing L-proline, the method comprising: culturing the strain described above to produce L-proline.

In another preferred embodiment, the method further comprises a step of isolating L-proline from a fermentation broth.

In a fourth aspect of the present disclosure, provided is a method for constructing a strain for producing L-proline, the method comprising enhancing the activity of the following polypeptide having an L-proline efflux function in the strain:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function.

In another preferred embodiment, the polypeptide is a polypeptide derived from a polypeptide having an amino acid sequence set forth in SEQ ID NO: 1 or 2, is formed by adding one or several, preferably 1-20, more preferably 1-15, more preferably 1-10, more preferably 1-3, and most preferably 1 amino acid residue at either or both ends of the amino acid sequence set forth in SEQ ID NO: 1 or 2, and has an L-proline efflux function.

In another preferred embodiment, the polypeptide has an amino acid sequence as set forth in SEQ ID NO: 1 or SEQ ID NO: 2.

In another preferred embodiment, the strain is a bacterium.

In another preferred embodiment, the strain is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067.

In another preferred embodiment, glutamate kinase in the bacterium is not subjected to feedback inhibition by L-proline or is subjected to attenuated feedback inhibition by L-proline.

In another preferred embodiment, the activity of glutamate kinase and/or glutamate-semialdehyde dehydrogenase and/or pyrroline-5-carboxylate reductase in the bacterium is enhanced.

In a fifth aspect of the present disclosure, provided is a host cell in which the activity of a polypeptide having an L-proline efflux function is attenuated, wherein the polypeptide having the activity of the L-proline efflux function is:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of the L-proline efflux function; or
C) a polypeptide having higher than 90%, 95%, 96%, 97%, 98%, or 99% homology to the polypeptide of A) and derived from *Corynebacterium* with the activity of an L-proline efflux function.

In another preferred embodiment, the host cell is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067.

In another preferred embodiment, glutamate kinase in the host cell is not subjected to feedback inhibition by L-proline or is subjected to attenuated feedback inhibition by L-proline.

In another preferred embodiment, the activity of a proline hydroxylase in the host cell is enhanced.

In another preferred embodiment, the proline hydroxylase is *trans*-proline-4-hydroxylase having a nucleotide sequence as set forth in SEQ ID NO: 5.

In another preferred embodiment, the activity of glutamate kinase and/or glutamate-semialdehyde dehydrogenase and/or pyrroline-5-carboxylate reductase in the bacterium is enhanced.

In a sixth aspect of the present disclosure, provided is a method for producing hydroxyproline such as *trans*-4-hydroxy-L-proline and the like, the method comprising: culturing the host cell described above to produce hydroxyproline such as *trans*-4-hydroxy-L-proline and the like. In another preferred embodiment, the method further comprises a step of isolating hydroxyproline such as *trans*-4-hydroxy-L-proline and the like from a fermentation broth.

In a seventh aspect of the present disclosure, provided is a method for constructing a strain for producing hydroxyproline such as *trans*-4-hydroxy-L-proline and the like, wherein the method comprises:
(1) attenuating the activity of the following polypeptide having an L-proline efflux function in the strain, wherein the polypeptide having the activity of the L-proline efflux function is:
   A) a polypeptide having an amino acid sequence set forth in SEQ ID NO: 1 or SEQ ID NO: 2; or
   B) a polypeptide having some amino acids added or deleted at either or both ends of the polypeptide of A) but still having the activity of the L-proline efflux function; or
   C) a polypeptide with homology higher than 90%, 95%, 96%, 97%, 98%, or 99% with the polypeptide of A) and derived from *Corynebacterium* with the activity of an L-proline efflux function;
(2) relieving or attenuating feedback inhibition by L-proline on glutamate kinase; and
(3) introducing a gene encoding a proline hydroxylase into the strain obtained in step (2).

In another preferred embodiment, the activity of glutamate kinase and/or glutamate-semialdehyde dehydrogenase and/or pyrroline-5-carboxylate reductase in the bacterium is enhanced.

In another preferred embodiment, the proline hydroxylase is *trans*-proline-4-hydroxylase having a nucleotide sequence as set forth in SEQ ID NO: 5.

The beneficial effects of the present disclosure are as follows:
1. In the present disclosure, after screening, ThrE, a threonine efflux protein, is found to be an L-proline efflux protein of *Corynebacterium glutamicum.* Production bacteria for L-proline, SZCgP1 and SZCgP3, are constructed based on strains ATCC13869 and ATCC13032 of *Corynebacterium glutamicum,* and overexpression of ThrE in the two strains can increase the yield of L-proline by 1.57 times and 2.56 times, respectively. Therefore, the present disclosure can be used in practice for producing L-proline by bacterial fermentation, is convenient to popularize and apply, and has considerable industrial application value.
2. In the present disclosure, ThrE is also used in the production of hydroxyproline such as *trans*-4-hydroxy-L-proline and the like, and knockout of *thrE* gene remarkably reduces the yield of extracellular L-proline by-products and increases the ratio of hydroxyproline such as *trans*-4-hydroxy-L-proline and the like to L-proline, helping to obtain hydroxyproline such as *trans*-4-hydroxy-L-proline and the like with higher purity. The knockout of *thrE* gene is of great significance to the production of hydroxyproline products such as *trans*-4-hydroxy-L-proline and the like.
3. The present disclosure provides a novel approach for constructing a hydroxyproline-producing strain by knocking out the L-proline efflux protein in the strain. This allows for an increase in the intracellular concentration of L-proline, which serves as a substrate for hydroxylation by proline hydroxylase. As a result, the intracellular L-proline is converted into hydroxyproline, thereby offering valuable insights for the development of higher-yielding hydroxyproline-producing strains in the field of biotechnology.

### BRIEF DESCRIPTION OF THE DRAWINGS

FIG. 1 shows a plasmid map of pCas9gRNA-ccdB;
FIG. 2 shows a plasmid map of *p*EC-*thrE*1; and
FIG. 3 shows a plasmid map of pEC*-thrE*2*.*

### DETAILED DESCRIPTION

The technical solutions of the present disclosure will be further illustrated in detail with reference to the following specific examples. It will be appreciated that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products or can be prepared using known methods. Where specific conditions are not indicated in experimental methods in the following examples, conventional conditions such as those described in Sambrook et al., Molecular Cloning: A Laboratory Manual (New York: Cold Spring Harbor Laboratory Press, 1989) or conditions recommended by the manufacturer are followed.

### Definitions and Description:

The term "polypeptide having an L-proline efflux function" used herein refers to a protein or a polypeptide that can excrete L-proline in a cell to the outside of the cell, which may be a protein set forth in SEQ ID NO: 1 or SEQ ID NO: 2, or a protein or a polypeptide that is obtained by mutating SEQ ID NO: 1 or SEQ ID NO: 2 and still has an L-proline efflux function.

The terms "protein", "polypeptide" and "peptide" used herein are used interchangeably, have the meanings commonly understood by one of ordinary skill in the art, and refer to amino acid polymers of any length. The polymers may be linear or branched. They may comprise modified amino acids and may be interrupted by non-amino acids. The terms also encompass amino acid polymers that have been modified (e.g., disulfide bond formation, glycosylation, lipidation, acetylation, phosphorylation, or any other operation, such as conjugation with a labeling component).

The term "fusion protein" used herein refers to a protein obtained by fusing the mutant protein with a protein tag. The protein tag can be located at the N-terminus of the mutant protein or at the C-terminus of the mutant protein. The mutant protein and the protein tag may also have a spacer amino acid residue therebetween, and specifically may have fewer than 10 spacer amino acid residues therebetween.

The term "host cell" herein means any type of cell that is susceptible to transformation, transfection, transduction, and the like using the polypeptide having the activity of the L-proline efflux function of the present disclosure, a polynucleotide encoding the polypeptide, or an expression vector. The term "recombinant microorganism" encompasses host cells which differ from parent cells after introduction of a transcription initiation element or a recombinant expression vector, specifically, the recombinant host cells are achieved by transformation. For example, suitable host cells for use in the present disclosure include, but are not limited to, *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067.

The term "transformation" herein has the meaning generally understood by those skilled in the art, i.e., the process of introducing exogenous DNA into a host. Methods for the transformation include any method for introducing a nucleic acid into a cell, including, but not limited to, electroporation, calcium phosphate precipitation, calcium chloride (CaCl₂) precipitation, microinjection, polyethylene glycol (PEG) method, DEAE-dextran method, cationic liposome method, and lithium acetate-DMSO method.

The culture of the host cell herein may be performed according to a conventional method in the art, including, but not limited to, well plate culture, shake-flask culture, batch culture, continuous culture, fed-batch culture, and the like, and various culture conditions such as temperature, time, pH of a medium, and the like may be appropriately adjusted according to actual circumstances.

The terms "comprise", "have", "include", or "contain" used herein are intended to be inclusive or open-ended and do not exclude additional, unspecified elements or method steps. The term "about" used herein means that a value includes the standard deviation of error for the device or method used to determine the value.

The term "or" used herein is defined as merely an alternative and "and/or", but unless it is explicitly stated to be only an alternative or mutually exclusive between alternatives, the term "or" in the claims means "and/or".

The selected/optional/preferred "numerical range" used herein includes both numerical endpoints at both ends of the range and all natural numbers covered between the numerical endpoints with respect to the aforementioned numerical endpoints.

The terms "wild-type" and "naturally occurring" used herein refer to an object that can be found in nature. For example, a polypeptide or polynucleotide sequence that is present in an organism, that can be isolated from a source in nature, and that has not been intentionally modified by humans in the laboratory, is naturally occurring.

The term "amino acid mutation" or "nucleotide mutation" used herein includes "substitution, repetition, deletion, or addition of one or more amino acids or nucleotides". In the present disclosure, the term "mutation" refers to an alteration in nucleotide sequence or amino acid sequence. In a specific embodiment, the term "mutation" refers to "substitution".

The term "native state" used herein refers to the activity of a polypeptide in a microorganism in an unmodified state, i.e., the activity in the native state.

The term "having the activity of an L-proline efflux function" used herein has the same or similar meaning as conventionally understood by those skilled in the art. It means that the amino acid sequence of a fragment is a portion of the amino acid sequence of an intact protein or polypeptide, and has the same or similar function or activity as the intact protein or polypeptide. Specifically, in the present disclosure, it indicates any amino acid fragment obtained from the ThrE protein of the present disclosure and having an L-proline efflux function.

It is known to those skilled in the art that, for mutating a wild-type polypeptide in order to improve the activity, it is more important to find a site that can achieve the desired purpose.

Therefore, based on the teachings of the present disclosure, a person skilled in the art will mutate the amino acid sequence set forth in SEQ ID NO: 1 or 2 and test the mutant for the relevant activity. In addition, it is not difficult for one of ordinary skill in the art to know that altering a few amino acid residues in certain regions of a polypeptide, e.g., in non-critical regions, does not substantially alter biological activity. For example, the sequence obtained by properly replacing some amino acids does not affect its activity (see Watson et al., Molecular Biology of The Gene, fourth edition, 1987, The Benjamin/Cummings Pub. Co. P224). Therefore, one of ordinary skill in the art would be able to implement such replacements and ensure that the resulting molecule retains the desired biological activity.

Therefore, it is apparent that further mutations of the L-proline efflux protein of the present disclosure result in further mutants that still have corresponding functions and activities. For example, it is well known to those skilled in the art that adding or removing several amino acid residues, e.g., preferably 1-20, more preferably 1-15, more preferably 1-10, more preferably 1-3, and most preferably 1 amino acid residue, to or from either end of a polypeptide does not affect the function of the resulting mutant. For example, for ease of purification, a skilled person usually makes the resulting protein have a 6×His tag at either end, and such protein has the same function as a protein that does not have the 6×His tag. Therefore, the present disclosure shall include conservative mutations obtained based on the present disclosure.

The term "conservative mutation" refers to a mutation that can normally maintain the function of a protein. A representative example of the conservative mutation is a conservative replacement.

As used in the present disclosure, the term "conservative replacement" relates to the replacement of an amino acid residue with an amino acid residue having a similar side chain. Families of amino acid residues having similar side chains have been defined in the art and include those having basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched chains (e.g., threonine, valine, and isoleucine), and aromatic side chains (e.g., tyrosine, phenylalanine, tryptophan, and histidine).

As used in the present disclosure, the "conservative replacement" typically refers to the substitution of one type of amino acid at one or more sites in a protein. Such substitution may be conservative. Examples of replacements regarded as conservative replacements include replacement of Ala with Ser or Thr, replacement of Arg with Gln, His or Lys, replacement of Asn with Glu, Gln, Lys, His or Asp, replacement of Asp with Asn, Glu or Gln, replacement of Cys with Ser or Ala, replacement of Gln with Asn, Glu, Lys, His, Asp or Arg, replacement of Glu with Gly, Asn, Gln, Lys or Asp, replacement of Gly with Pro, replacement of His with Asn, Lys, Gln, Arg or Tyr, replacement of Ile with Leu, Met, Val or Phe, replacement of Leu with Ile, Met, Val or Phe, replacement of Lys with Asn, Glu, Gln, His or Arg, replacement of Met with Ile, Leu, Val or Phe, replacement of Phe with Trp, Tyr, Met, Ile, or Leu, replacement of Ser with Thr or Ala, replacement of Thr with Ser or Ala, replacement of Trp with Phe or Tyr, replacement of Tyr with His, Phe or Trp, and replacement of Val with Met, Ile or Leu. In addition, the conservative mutations also include naturally occurring mutations caused by individual differences, differences in strain and species, and the like from which genes are derived.

The term "corresponding to" used herein has the meaning commonly understood by one of ordinary skill in the art. Specifically, the term "corresponding to" refers to a position in a sequence corresponding to a specified position in another sequence after alignment of the two sequences by homology or sequence identity. Thus, for example, in terms of "an amino acid residue corresponding to position 40 of the amino acid sequence set forth in SEQ ID NO: 1", if a 6×His tag is added to one end of the amino acid sequence set forth in SEQ ID NO: 1, then position 40 of the resulting mutant corresponding to the amino acid sequence set forth in SEQ ID NO: 1 may be position 46.

In a specific embodiment, the homology or sequence identity may be no less than 90%, preferably no less than 95%, and more preferably 96%, 97%, 98%, or 99% homology.

Methods for determining sequence homology or identity well known to one of ordinary skill in the art include, but are not limited to: Computational Molecular Biology, Ed. Lesk, A.M., Oxford University Press, New York, 1988; Biocomputing: Informatics and Genome Projects, Ed. Smith, D.W., Academic Press, New York, 1993; Computer Analysis of Sequence Data, Part One, Ed. Griffin, A.M. and Griffin, H.G., Humana Press, New Jersey, 1994; Sequence Analysis in Molecular Biology, von Heinje, G., Academic Press, 1987; Sequence Analysis Primer, Ed. Gribskov, M. and Devereux, J., M Stockton Press, New York, 1991; and Carillo, H. and Lipman, D., SIAM J. Applied Math., 48:1073 (1988). A preferred method for determining identity is to obtain the greatest match between the sequences tested. Methods for determining identity are compiled in publicly available computer programs. Preferred computer program methods for determining identity between two sequences include, but are not limited to: GCG program package (Devereux, J. et al., 1984), BLASTP, BLASTN, and FASTA (Altschul, S, F. et al., 1990). The BLASTX program is publicly available from NCBI and other sources (BLAST Manual, Altschul, S. et al., NCBI NLM NIH Bethesda, Md.20894; Altschul, S. et al., 1990). The well-known Smith Waterman algorithm can also be used to determine identity.

Unless defined otherwise, or clear from the background, all technical and scientific terms used in the present disclosure have the same meaning as commonly understood by one of ordinary skill in the art to which the present disclosure belongs.

### Example 1. Screening of L-Proline Efflux Proteins of Corynebacterium Glutamicum in Corynebacterium Glutamicum ATCC13869

The inventors screened L-proline efflux proteins of *Corynebacterium glutamicum* by a whole genome-scale membrane transporter inhibition library. Firstly, the cas9 gene of the pCas9 plasmid was mutated with D10A and H840A, and the Bsa I restriction enzyme site in the plasmid backbone was removed, resulting in the pdCas9 plasmid (LIU, Jiao, et al. Development of a CRISPR/Cas9 genome editing toolbox for Corynebacterium glutamicum. Microbial cell factories, 2017, 16.1: 205.). Subsequently, a gRNA-*ccdB* expression cassette was amplified from a pnCas9(D10A)-AID-gRNA-*ccdB*^{TS} plasmid (WANG, Yu, et al. Expanding targeting scope, editing window, and base transition capability of base editing in Corynebacterium glutamicum. Biotechnology and bioengineering, 2019, 116: 3016-3029) and cloned into the same position of pdCas9 to obtain pdCas9gRNA-*ccdB* as a CRISPRi plasmid that could be efficiently constructed and had a sequence as set forth in SEQ ID NO: 3. The inhibition efficiency of the system could reach 96%. Based on the above ingenious design, for the inhibition of the target gene, only a sgRNA with a 20 bp target DNA binding region was required to be designed, then two complementary primers of 24 bp were synthesized and annealed to form a double-stranded DNA fragment, and an inhibition plasmid of the target gene was constructed by Golden Gate cloning. The inventors also constructed a control plasmid pdCas9gRNA-control without the 20 bp DNA binding region. Whole genome-scale protein sequences of *Corynebacterium glutamicum* ATCC13869 were used in a TransportDB database for membrane transporter protein prediction to obtain 397 membrane transporters on the whole genome scale. Thereafter, inhibition plasmids of genes of the 397 membrane transporters were constructed based on the CRISPRi system, and the inhibition library plasmids and the control plasmid were separately introduced into a bacterium for producing L-proline, SZCgP1 *(Corynebacterium glutamicum* ATCC13869, in which a G149D mutation of *proB* gene was introduced, with a codon mutation from GGT to GAT, see CN101084312A for details), so as to obtain inhibition library strains. Finally, all of the strains were evaluated for the specific productivity of L-proline by fermentation on 96-well plates and genes with decreased specific productivity of L-proline after inhibition were potential L-proline efflux protein genes. Based on the above screening, the inventors found that the inhibition of *thrE* (Cgl2622, which has been confirmed in the literature as an L-threonine efflux protein) gene resulted in a significant decrease in the specific productivity of L-proline. Primers for the construction of the CRISPRi plasmids of the *thrE* gene were *thrE*-F and *thrE*-R (Table 1). The control strain was SZCgP1(pdCas9gRNA-control), the inhibition plasmid of the *thrE* gene was pdCas9gRNA-*thrE*, and the inhibition strain of the *thrE* gene was SZCgP1(pdCas9gRNA-*thrE*).

**Table 1**

| Primer | Nucleotide sequence | SEQ ID NO. |
|---|---|---|
| *thrE-F* | TTCACACTGCTCGAACTTGTACCT | 6 |
| *thrE-R* | AAACAGGTACAAGTTCGAGCAGTG | 7 |

To further confirm the effect of the *thrE* gene inhibition on L-proline production, evaluations were performed by fermentation using a 24-well deep-well plate. The seed culture medium contained the following ingredients (g/L): 5 g/L glucose, 1 g/L yeast powder, 3 g/L soybean peptone, 3 g/L urea, 0.5 g/L succinic acid, 1 g/L K₂HPO₄·3H₂O, 0.1 g/L MgSO₄·7H₂O, 0.01 mg/L biotin, 0.1 mg/L vitamin B1, and 20 g/L MOPS. The fermentation culture medium contained the following ingredients: 80 g/L glucose, 1 g/L yeast powder, 1 g/L soybean peptone, 1 g/L NaCl, 1 g/L ammonium sulfate, 6 g/L urea, 1 g/L K₂HPO₄·3H₂, 0.45 g/L MgSO₄·7H₂O, 0.05 g/L FeSO₄·7H₂O, 0.4 mg/L biotin, 0.1 mg/L vitamin B1, and 40 g/L MOPS, and the initial pH was 7.2. Firstly, the strains were inoculated into a seed culture medium containing 15 µg/mL chloramphenicol and cultured for 8 h. The cultures were inoculated as seeds into a 24-well plate containing 15 µg/mL chloramphenicol and a 0.03 mM IPTG fermentation culture medium. The volume of the medium was 800 µL, and the inoculation amount was controlled with the initial OD₆₀₀ being 0.03 (as detected by a microplate reader). The seeds were cultured at 30 °C for 21 h, with the rotation speed of a plate shaker being 800 rpm. The experiment for each strain was performed in triplicate. After fermentation, OD₆₀₀ and the yield of L-proline were detected. The detection of L-proline was performed as follows: The fermentation solution was diluted with 3% (W/V) sulfosalicylic acid to a proper concentration. 1 mL of the dilution was taken, and 1 mL of an acid-ninhydrin complex (1.25 g of ninhydrin dissolved in 30 mL of glacial acetic acid and 20 mL of 6M H₃PO₄ by heating at 70 °C) and 1 mL of glacial acetic acid were added. The mixture was reacted in a boiling water bath at 100 °C for 45 min. After cooling, OD₅₂₀ was determined. A standard curve was plotted by adopting L-proline at concentrations of 0-100 mg/L, and the concentrations of the samples to be tested were calculated according to the standard curve. The results are shown in Table 2, indicating that the yield and specific productivity of L-proline of the strains decreased by 63% and 49%, respectively, after the inhibition of *thrE* gene expression.

**Table 2. Effect of inhibition of thrE gene expression on yield and specific productivity of L-proline**

| Strain | OD₆₀₀ | Yield of L-proline (g/L) | Specific productivity of L-proline (g/L/ OD₆₀₀) |
|---|---|---|---|
| SZCgP 1 (pdCas9gRNA-control) | 13.60±1.42 | 5.05±0.50 | 0.37±0.00 |
| SZCgP 1 (pdCas9gRNA-*thrE*) | 9.91±0.64 | 1.87±0.29 | 0.19±0.03 |

### Example 2. Identification of L-Proline Efflux Proteins of Corynebacterium Glutamicum in Corynebacterium Glutamicum ATCC13869

### (1) Construction of knockout, complementation and overexpression strains of thrE gene in SZCgP1 strains

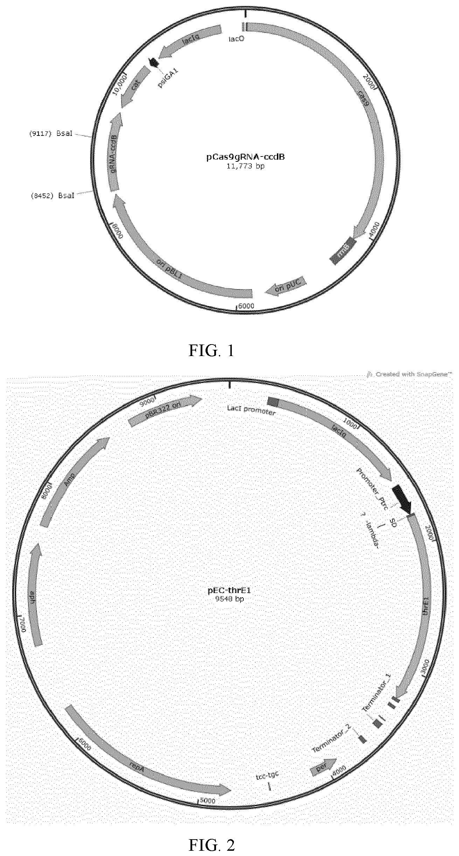

The inventors constructed knockout mutants based on CRISPR/Cas9 genome editing technology. Using a pCas9 plasmid (LIU, Jiao, et al. Development of a CRISPR/Cas9 genome editing toolbox for Corynebacterium glutamicum. Microbial cell factories, 2017, 16.1: 205.) as a template and *cas*9-1/*cas*9-2 as primers, a lacO mutation (a mutation from TGTGTGGAATTGTGAGCGGATAACAATTTCACACA to TGTGTGGAATTGTGAGCGCTCACAATTTCACACA) and an RBS mutation (a mutation from AAAGGAGTTGAGA to AAAGGCACCCGAT) as manipulation elements were introduced on the primers, and a fragment containing *cas9* was amplified. Thereafter, a plasmid backbone fragment containing a gRNA-*ccdB* expression cassette and a temperature-sensitive replication origin was amplified by using a pnCas9(D10A)-AID-gRNA-*ccdB*^{TS} plasmid (WANG, Yu, et al. Expanding targeting scope, editing window, and base transition capability of base editing in Corynebacterium glutamicum. Biotechnology and bioengineering, 2019, 116: 3016-3029) as a template and gRNA-1/gRNA-2 as primers. The above 2 fragments were cloned and ligated by a one-step recombination kit from Vazyme to obtain a pCas9gRNA-*ccdB* plasmid that could be efficiently constructed and had a plasmid map as shown in FIG. 1 and a sequence as set forth in SEQ ID NO: 4. According to the reported genome sequence of *Corynebacterium glutamicum* ATCC13869, upstream and downstream homologous arms were amplified by using ATCC13869 genome as a template with *thrE*-1/ *thrE*-2 and *thrE*-3/ *thrE*-4 as primers, respectively. 3 plasmid backbone fragments were amplified by using a pCas9gRNA-*ccdB* plasmid as a template with *cas*9-3/*cas*9-4, *cas*9-5/*cas*9-6, and gRNA-3/gRNA-4 as primers, respectively, and a gRNA with 20bp target DNA binding region was also introduced. The above 5 fragments were cloned and ligated by a one-step recombination kit from Vazyme to obtain a pCas9gRNA-*thrE* plasmid. SZCgP1 strain was prepared to be competent, and about 2 µg of the pCas9gRNA-*thrE* plasmid was electrotransformed. 1 mL of a TSB culture medium pre-heated at 46 °C was added, and the heat shock was performed at 46 °C for 6 min. The mixture was incubated at 30 °C for 3 h, applied onto a TSB plate containing 15 µg/mL chloramphenicol and 0.05 mM IPTG, and cultured for 2 days. A correct mutant verified by PCR and sequencing was named as SZCgP2 strain.

To construct the overexpression plasmid, the thrE gene was cloned onto the pEC-ccdB plasmid, which is derived from the pEC-XK99E plasmid. The pEC-*ccdB* plasmid was digested using a *Bsa* I enzyme. According to the reported genome sequence of *Corynebacterium glutamicum* ATCC13869, a fragment of *thrE*1 gene was amplified with *thrE-5*/*thrE-6* by using the genome of ATCC13869 as a template, and an amino acid sequence of a protein encoded by the *thrE*1 gene is as set forth in SEQ ID NO: 1. The two segments were cloned and ligated by a one-step recombination kit from Vazyme to obtain a pEC-*thrE*1 plasmid having a plasmid map as shown in FIG. 2. A control plasmid pEC-control, in which no gene was cloned, was also constructed. The pEC-*thrE*1 plasmid was transformed into the SZCgP2 strain to obtain a complementation strain SZCgP2(pEC-*thrE*1). The pEC-*thrE*1 and pEC-control plasmids were transformed into SZCgP1 to obtain an overexpression strain SZCgP1(pEC-*thrE*1) and a control strain SZCgP1(pEC-control), respectively.

**Table 3**

| Primer | Nucleotide sequence | SEQ ID NO. |
|---|---|---|
| cas9-1 | GCGCTCACAATTTCACACAGGAAACAGAATTAAT TAAGCTTAAAGGCACCCGATATGGATAAGAAATA CTCAATAGGC | 8 |
| cas9-2 | TCAGTCACCTCCTAGCTGACTCAAATC | 9 |
| gRNA-1 | GTCAGCTAGGAGGTGACTGAAGCTTGGCTGTTTT GGCGGATG | 10 |
| gRNA-2 | CTGTGTGAAATTGTGAGCGCTCACAATTCC | 11 |
| Cas9-3 | TCGAAGGGCACCAATAACTGC | 12 |
| Cas9-4 | CTTTTACTTTCACCAGCGTTTCTG | 13 |
| Cas9-5 | AACGCTGGTGAAAGTAAAAGATGC | 14 |
| Cas9-6 | GAATGGAACGGATCAAACGGTGAATTACACTGTA CCTGTTGCGTC | 15 |
| gRNA-3 | CCGTTTGATCCGTTCCATTCGTTTTAGAGCTAGAA ATAGCAAG | 16 |
| gRNA-4 | CAACCTGCCATCACGAGATTTTC | 17 |
| *thrE*-1 | AATCTCGTGATGGCAGGTTGACGGGAGCCTAAGT GAAATG | 18 |
| *thrE*-2 | CCTTGGTAAATGCACGGTATGGGTCAGTGAGATC AATCGGG | 19 |
| *thrE*-3 | CCATACCGTGCATTTACCAAGG | 20 |
| *thrE*-4 | CAGTTATTGGTGCCCTTCGACTTTGTGGTCTTCTT TGCGG | 21 |
| *thrE*-5 | ACAGGCCAAAGGAGTTGAGAATGTTGAGTTTTGC GACCC | 22 |
| *thrE*-6 | CCAAGCTTGCATGCCTGCAGTTACCTTTTATTACC GAATC | 23 |

(2) Determination of intracellular and extracellular L-proline concentrations of knockout, complementation and overexpression strains of *thrE* gene by shake-flask fermentation To further confirm whether the *thrE* gene is an L-proline efflux protein, intracellular and extracellular L-proline concentrations of knockout, complementation and overexpression strains were determined using shake-flask fermentation. The seed culture medium contained the following ingredients (g/L): 5 g/L glucose, 1 g/L yeast powder, 3 g/L soybean peptone, 3 g/L urea, 0.5 g/L succinic acid, 1 g/L K₂HPO₄·3H₂O, 0.1 g/L MgSO₄·7H₂O, 0.01 mg/L biotin, 0.1 mg/L vitamin B1, and 20 g/L MOPS. The fermentation culture medium contained the following ingredients: 80 g/L glucose, 1 g/L yeast powder, 1 g/L soybean peptone, 1 g/L NaCl, 1 g/L ammonium sulfate, 6 g/L urea, 1 g/L K₂HPO₄·3H₂, 0.45 g/L MgSO₄·7H₂O, 0.05 g/L FeSO₄·7H₂O, 0.4 mg/L biotin, 0.1 mg/L vitamin B1, and 40 g/L MOPS, and the initial pH was 7.2. Firstly, the strains were inoculated into a seed culture medium and cultured for 8 h. The cultures were inoculated as seeds into 250 mL triangular flasks containing a fermentation culture medium, the volume of the medium being 25 mL. 25 µg/mL kanamycin and 0.05 mM IPTG were added as needed, and the inoculation amount was controlled with the initial OD₆₀₀ being 0.1 (as determined by a spectrophotometer). The mixture was cultured at 30 °C for 21 h, with the rotation speed of a shaker being 2200 rpm. The experiment for each strain was performed in triplicate. Samples were taken in the fermentation process to determine OD₆₀₀, intracellular L-proline concentration and extracellular L-proline concentration.

The intracellular and extracellular L-proline concentrations were determined as follows: 400 µL of silicone oil was added into a 1.5 mL centrifuge tube in advance, and 400 µL of the fermented bacterial solution was quickly pipetted and then slowly added into the tube. The mixture was centrifuged at 4 °C and 12000 rpm for 10 min, and the supernatant was obtained as an extracellular sample. The bacteria precipitate at the bottom of the centrifuge tube was cut off and placed in a 1.5 mL centrifuge tube. A proper amount of 3% sulfosalicylic acid was added according to the amount of the bacteria. The mixture was boiled at 100 °C for 20 min and centrifuged at 12000 rpm for 10 min, and the supernatant was obtained as an intracellular sample. The detection of L-proline was performed as described in Example 1, and the intracellular L-proline concentration was calculated based on the volume of *Corynebacterium glutamicum* reported in the literature.

The results of the determination of the intracellular and extracellular L-proline concentrations of each strain in the fermentation process are as shown in Tables 4 and 5. The extracellular L-proline concentration of the *thrE* gene-knockout strain SZCgP2 was significantly lower than that of the starting strain SZCgP1 throughout the fermentation process, and the intracellular L-proline concentration of the knockout strain was slightly higher than that of the starting strain SZCgP1 in the early stage (3-9 h) of the fermentation. The extracellular L-proline concentration of the complementation strain SZCgP2(pEC-*thrE*1) was significantly higher than that of the starting strain SZCgP1 throughout the fermentation process, and the intracellular L-proline concentration of the complementation strain was significantly lower than that of the starting strain during the rapid acidogenic phase (3-15 h). The extracellular L-proline concentration of the overexpression strain SZCgP1(pEC-*thrE*1) was significantly higher than that of the control strain SZCgP1(pEC-control) throughout the fermentation process, and the intracellular L-proline concentration of the overexpression strain was significantly lower than that of the control strain SZCgP1(pEC-control) throughout the fermentation process (especially during the rapid acidogenic phase). Based on the above results, it was indicated that ThrE is a proline efflux protein. The overexpression of ThrE in the SZCgP1 strain could increase the yield of L-proline, and the overexpression strain SZCgP1(pEC-*thrE*1) showed a 1.57-fold increase in the yield of extracellular L-proline at 21 h relative to the control bacterium SZCgP1(pEC-control) introduced with an empty plasmid, indicating that the overexpression of the L-proline efflux protein gene is of great significance in the modification of a strain producing L-proline.

**Table 4. Intracellular L-proline concentrations of knockout, complementation and overexpression strains of thrE gene in Corynebacterium glutamicum ATCC13869**

| Time | Intracellular L-proline concentration (mM) | | | | |
|---|---|---|---|---|---|
| | SZCgP1 | SZCgP2 | SZCgP2 (pEC-*thrE*1) | SZCgP1 (pEC-*thrE*1) | SZCgP1 (pEC-control) |
| 3 h | 151±17 | 213±21 | 83±1 | 67±10 | 123±7 |
| 6 h | 192±17 | 239±13 | 72±3 | 73±2 | 221±61 |
| 9 h | 296±18 | 315±15 | 116±19 | 106±10 | 246±37 |
| 12 h | 287±19 | 256±14 | 112±8 | 107±5 | 228±21 |
| 15 h | 206 ±37 | 235±15 | 101±2 | 92±3 | 228±11 |
| 18 h | 103±5 | 110±6 | 105±3 | 95±2 | 159± 9 |
| 21 h | 76±6 | 70±1 | 111±4 | 105±5 | 108±9 |

**Table 5. Extracellular L-proline concentrations of knockout, complementation and overexpression strains of thrE gene in Corynebacterium glutamicum ATCC13869**

| Time | Extracellular L-proline concentration (g/L) | | | | |
|---|---|---|---|---|---|
| | SZCgP1 | SZCgP2 | SZCgP2 (pEC-*thrE*1) | SZCgP1 (pEC-*thrE*1) | SZCgP1 (pEC-control) |
| 3 h | 0.05±0.00 | 0.03±0.00 | 0.08±0.00 | 0.08±0.00 | 0.04±0.00 |
| 6 h | 0.15±0.00 | 0.03±0.00 | 0.32±0.03 | 0.30±0.02 | 0.12±0.01 |
| 9 h | 0.65±0.02 | 0.20±0.04 | 1.40±0.10 | 1.98±0.26 | 0.47±0.02 |
| 12 h | 1.49±0.37 | 0.47±0.00 | 3.14±0.35 | 3.09±0.14 | 0.87±0.09 |
| 15 h | 5.41±0.12 | 2.42±0.05 | 11.61±0.96 | 11.77±1.14 | 4.40±0.16 |
| 18 h | 5.41±1.09 | 3.40±0.69 | 17.01±0.96 | 16.40±4.23 | 5.34±1.48 |
| 21 h | 5.56±0.41 | 2.71±0.29 | 17.87±2.90 | 16.22±4.31 | 6.32±0.31 |

### Example 3. Use of L-Proline Efflux Protein in Production of L-Proline in Corynebacterium Glutamicum ATCC13032 Strain

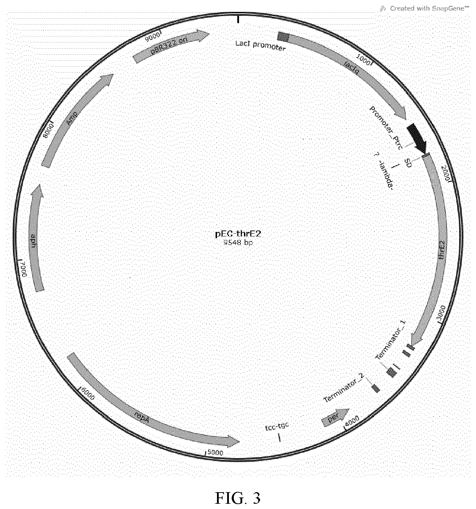

Firstly, a strain producing L-proline, SZCgP3, was constructed based on a *Corynebacterium glutamicum* ATCC13032 strain, i.e., a G149K mutation of *proB* gene was introduced, with a codon mutation from GGT to AAG (see CN101084312A). The construction of a ThrE overexpression strain was similar to that of a *Corynebacterium glutamicum* 13869 strain. Overexpression of *thrE* was performed on a pEC-*ccdB* plasmid, a derivative plasmid of pEC-XK99E plasmid, and the pEC*-ccdB* plasmid was digested using a *Bsa* I enzyme. A fragment of *thrE2* gene was amplified with *thrE-5*/*thrE-6* by using the genome of *Corynebacterium glutamicum* ATCC13032 as a template, and an amino acid sequence of a protein encoded by the *thrE*2 gene is as set forth in SEQ ID NO: 2. The two segments were cloned and ligated by a one-step recombination kit from Vazyme to obtain a pEC-*thrE*2 plasmid having a plasmid map as shown in FIG. 3. The pEC-*thrE*2 and pEC-control plasmids were transformed into SZCgP3 to obtain an overexpression strain SZCgP3(pEC-*thrE*2) and a control strain SZCgP3(pEC-control), respectively. The yield of extracellular L-proline of the above strains was evaluated by shake-flask fermentation, and the fermentation was performed as described in Example 2. The results are shown in Table 6, indicating that the overexpression of ThrE in *Corynebacterium glutamicum* SZCgP3 could increase the yield of L-proline, and the overexpression strain SZCgP3(pEC-*thrE*2) showed a 2.56-fold increase in the yield of L-proline at 21 h relative to the control bacterium SZCgP3(pEC-control) introduced with an empty plasmid, further indicating that the overexpression of the L-proline efflux protein gene in *Corynebacterium glutamicum* is of great significance in the production of L-proline.

**Table 6. Yield of extracellular L-proline of thrE gene overexpression strain in Corynebacterium glutamicum ATCC13032**

| Time | Yield of extracellular L-proline (g/L) | |
|---|---|---|
| | SZCgP3(pEC-control) | SZCgP3(pEC-*thrE*2) |
| 3 h | 0.02±0.00 | 0.03±0.00 |
| 6 h | 0.05±0.00 | 0.17±0.01 |
| 9 h | 0.22±0.02 | 0.80±0.09 |
| 12 h | 0.59±0.01 | 1.56±0.07 |
| 15 h | 1.00±0.03 | 3.56±0.22 |
| 18 h | 3.85±0.36 | 10.55±0.36 |
| 21 h | 3.81±0.47 | 13.56±1.26 |

### Example 4. Use of L-Proline Efflux Protein in Production of Hydroxyproline

Firstly, an expression plasmid pEC-mip4h was constructed for expressing trans-proline-4-hydroxylase, based on the pEC-XK99E plasmid, as described in Patent No. CN201710661945.1. The sequence of trans-proline-4-hydroxylase is provided as SEQ ID NO: 5.. The pEC*-mip4h* plasmid and the pEC-XK99E control plasmid were transformed into an SZCgP1 strain to obtain an overexpression strain SZCgP1(pEC-*mip4h*) and a control strain SZCgP1(pEC-XK99E), respectively, and were transformed into an SZCgP2 strain to obtain an overexpression strain SZCgP2(pEC-*mip4h*) and a control strain SZCgP2(pEC-XK99E), respectively.

The seed culture medium contained the following ingredients (g/L): 5 g/L glucose, 1 g/L yeast powder, 3 g/L soybean peptone, 3 g/L urea, 0.5 g/L succinic acid, 1 g/L K₂HPO₄·3H₂O, 0.1 g/L MgSO₄·7H₂O, 0.01 mg/L biotin, 0.1 mg/L vitamin B1, and 20 g/L MOPS. The fermentation culture medium contained the following ingredients: 80 g/L glucose, 1 g/L yeast powder, 1 g/L soybean peptone, 1 g/L NaCl, 1 g/L ammonium sulfate, 6 g/L urea, 1 g/L K₂HPO₄·3H₂O, 0.45 g/L MgSO₄·7H₂O, 0.2 g/L FeSO₄·7H₂O, 0.4 mg/L biotin, 0.1 mg/L vitamin B1, and 40 g/L MOPS, and the initial pH was 7.2. Firstly, the strains were inoculated into a seed culture medium and cultured for 8 h. The cultures were inoculated as seeds into a 24-well plate containing 800 µL of a fermentation culture medium, and the inoculation amount was controlled with the initial OD₆₀₀ being 0.1 (as detected by a microplate reader). The seeds were cultured at 30 °C for 18 h, with the rotation speed of a plate shaker being 800 rpm. The experiment for each strain was performed in triplicate. After fermentation, OD₆₀₀ and the yield of *trans*-4-hydroxy-L-proline and L-proline were detected. The detection of *trans*-4-hydroxy-L-proline was performed with reference to the China national standard GB/T 9695.23-2008, and the detection of L-proline was performed as described in Example 1. The results are shown in Table 7, indicating that the strains without expression of *trans*-proline-4-hydroxylase failed to produce *trans*-4-hydroxy-L-proline, and that the knockout of the L-proline efflux protein *thrE* gene could remarkably reduce the yield of extracellular L-proline by-products and increase the ratio of *trans*-4-hydroxy-L-proline to L-proline, helping to obtain *trans*-4-hydroxy-L-proline production with higher purity. It is expected that further enhancement of the activity of *trans*-proline-4-hydroxylase allows rapid transformation of intracellularly synthesized L-proline to *trans*-4-hydroxy-L-proline in the strain, and knockout of the L-proline efflux protein *thrE* gene also reduces the efflux of L-proline to the extracellular space, allowing the yield of *trans*-4-hydroxy-L-proline to be also increased. Therefore, the knockout of L-proline efflux protein *thrE* is of great significance to the production of *trans*-4-hydroxy-L-proline.

**Table 7. Effect of thrE gene knockout on the yield of extracellular trans-4-hydroxy-L-proline and L-proline**

| Strain | OD₆₀₀ | Yield of hydroxyproline (g/L) | Yield of proline (g/L) | Ratio of hydroxyproline to proline |
|---|---|---|---|---|
| SZCgP1(pEC-XK99E) | 16.47±0.94 | - | 4.25±0.35 | - |
| SZCgP1(pEC-*mip4h*) | 13.16±0.18 | 0.70±0.01 | 2.94±0.21 | 1:4.2 |
| SZCgP2(pEC-XK99E) | 15.52±0.82 | - | 1.68±0.08 | - |
| SZCgP2(pEC-*mip4h*) | 14.31±0.22 | 0.70±0.05 | 0.89±0.05 | 1:1.3 |

### Example 5. Use of L-Proline Efflux Protein in Production of Hydroxyproline

To further verify the use of the L-proline efflux protein in the production of other hydroxyproline, in the present disclosure, a plasmid for expressing *trans*-proline-3-hydroxylase was first constructed, that is, an expression plasmid pEC*-Ubp4h* for expressing the *trans*-proline-3-hydroxylase Ubp4h as shown in Patent No. CN110804596A was artificially synthesized based on a pEC-XK99E plasmid. The pEC-*Ubp4h* plasmid and the pEC-XK99E control plasmid were transformed into an SZCgP1 strain to obtain an overexpression strain SZCgP1(pEC-*Ubp4h*) and a control strain SZCgP1(pEC-XK99E), respectively, and were transformed into an SZCgP2 strain to obtain an overexpression strain SZCgP2(pEC-*Ubp4h*) and a control strain SZCgP2(pEC-XK99E), respectively. The ingredients in the medium and the culture of the strains were referred to Example 4. The detection of *trans*-3-hydroxy-L-proline was performed with reference to Patent No. CN110804596A, and the detection of L-proline was performed as described in Example 1. The result shows that the strains without expression of *trans*-proline-3-hydroxylase failed to produce *trans*-3-hydroxy-L-proline, and that the knockout of the L-proline efflux protein *thrE* gene could remarkably reduce the yield of extracellular L-proline by-products and increase the ratio of *trans*-3-hydroxy-L-proline to L-proline, helping to obtain *trans*-3-hydroxy-L-proline production with higher purity. That is, the knockout of L-proline efflux protein *thrE* is of great significance to the production of *trans*-3-hydroxy-L-proline.

Hydroxyproline has four natural stereoisomers, namely *trans*-4-hydroxy-L-proline, *cis*-4-hydroxy-L-proline, *trans*-3-hydroxy-L-proline, and *cis*-3-hydroxy-L-proline, and it is known to those skilled in the art that the four different hydroxyprolines are obtained by directly catalyzing L-proline with corresponding *trans*-proline-4-hydroxylase, *cis*-proline-4-hydroxylase, *trans*-proline-3-hydroxylase, and *cis*-proline-3-hydroxylase, respectively. Documents in the prior art have also reported that different hydroxyprolines can be produced by overexpressing different proline hydroxylases based on strains producing L-proline (Zhang F, Liu H, Zhang T, Pijning T, Yu L, Zhang W, Liu W, Meng X. Biochemical and genetic characterization of fungal proline hydroxylase in echinocandin biosynthesis. Appl Microbiol Biotechnol. 2018 Sep;102(18):7877-7890. Chen K, Pang Y, Zhang B, Feng J, Xu S, Wang X, Ouyang P. Process optimization for enhancing production of cis-4-hydroxy-L-proline by engineered Escherichia coli. Microb Cell Fact. 2017 Nov 22;16(1):210. Mori H, Shibasaki T, Yano K, Ozaki A. Purification and cloning of a proline 3-hydroxylase, a novel enzyme which hydroxylates free L-proline to cis-3-hydroxy-L-proline. J Bacteriol. 1997 Sep;179(18):5677-83). Therefore, for those skilled in the art, in Examples 4 and 5 of the present disclosure, it is demonstrated that the knockout of the L-proline efflux protein ThrE can increase intracellular L-proline concentration, and in the case that, based on this strain, the overexpression of *trans*-proline-4-hydroxylase can increase the yield of *trans*-4-hydroxy-L-proline, and the overexpression of *trans*-proline-3-hydroxylase can increase the yield of *trans*-3-hydroxy-L-proline; it is apparent to those skilled in the art that the overexpression of different proline hydroxylases based on the knockout of the L-proline efflux protein ThrE can reduce the yield of extracellular L-proline as by-products and increase the corresponding ratio of hydroxyproline to L-proline, i.e., the knockout of the L-proline efflux protein is of great significance to the production of all of the above 4 hydroxyprolines. The embodiments of the present disclosure have been described above. However, the present disclosure is not limited to the embodiments described above. Any modification, equivalent, improvement, and the like made without departing from the spirit and principle of the present disclosure shall fall within the protection scope of the present disclosure.

## Claims

1. Use of a polypeptide in producing L-proline or hydroxyproline, wherein the polypeptide is:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function;
preferably, the polypeptide has an amino acid sequence as set forth in SEQ ID NO: 1 or SEQ ID NO: 2.

2. A strain for producing L-proline, wherein the strain expresses the following polypeptide:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function;
preferably, the polypeptide has an amino acid sequence as set forth in SEQ ID NO: 1 or SEQ ID NO: 2;
preferably, the strain is a bacterium;
preferably, the strain is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067;
more preferably, glutamate kinase in the bacterium is not subjected to feedback inhibition by L-proline or is attenuated feedback inhibition by L-proline;
even more preferably, the activity of glutamate kinase and/or glutamate-semialdehyde dehydrogenase and/or pyrroline-5-carboxylate reductase in the bacterium is enhanced.

3. A method for producing L-proline, wherein the method comprises: culturing the strain of claim 2 to produce L-proline;
preferably, the method further comprises a step of isolating L-proline from a fermentation broth.

4. A method for constructing a strain for producing L-proline, wherein the method comprises enhancing the activity of the following polypeptide in the strain:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of an L-proline efflux function; or
C) a polypeptide having higher than 90%, 95%, 96%, 97%, 98%, or 99% homology to the polypeptide of A) and derived from *Corynebacterium* with the activity of an L-proline efflux function;
preferably, the polypeptide has an amino acid sequence as set forth in SEQ ID NO: 1 or SEQ ID NO: 2;
preferably, the strain is a bacterium;
preferably, the strain is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067;
more preferably, glutamate kinase in the bacterium is not subjected to feedback inhibition by L-proline or is subjected to an attenuated feedback inhibition by L-proline;
even more preferably, the activity of glutamate kinase and/or glutamate-semialdehyde dehydrogenase and/or pyrroline-5-carboxylate reductase in the bacterium is enhanced.

5. A host cell, wherein the activity of a polypeptide having an L-proline efflux function in the host cell is attenuated, wherein the polypeptide having the activity of the L-proline efflux function is:
A) a polypeptide having an amino acid sequence set forth in SEQ ID NO: 1 or SEQ ID NO: 2; or
B) a polypeptide having some amino acids added or deleted at either or both ends of the polypeptide of A) but still having the activity of the L-proline efflux function; or
C) a polypeptide having higher than 90%, 95%, 96%, 97%, 98%, or 99% homology to the polypeptide of A) and derived from *Corynebacterium* with the activity of an L-proline efflux function;
preferably, the host cell is selected from *Escherichia* and *Corynebacterium,* preferably *Escherichia coli* and *Corynebacterium glutamicum,* more preferably *Corynebacterium glutamicum,* and more particularly *Corynebacterium glutamicum* ATCC 13032, *Corynebacterium glutamicum* ATCC13869, *Corynebacterium glutamicum* B253, and *Corynebacterium glutamicum* ATCC 14067;
more preferably, glutamate kinase in the host cell is not subjected to feedback inhibition by L-proline or is subjected to an attenuated feedback inhibition by L-proline;
even more preferably, the activity of a proline hydroxylase in the host cell is enhanced;
even more preferably, the activity of glutamate kinase and/or glutamate-semialdehyde dehydrogenase and/or pyrroline-5-carboxylate reductase in the host cell is enhanced.

6. The host cell as claimed in claim 5, wherein the proline hydroxylase is *trans*-proline-4-hydroxylase having a nucleotide sequence as set forth in SEQ ID NO: 5.

7. A method for producing hydroxyproline, wherein the method comprises: culturing the host cell of claim 5 or 6 to produce hydroxyproline;
preferably, the method further comprises a step of isolating hydroxyproline from a fermentation broth.

8. A method for constructing a strain for producing hydroxyproline, wherein the method comprises:
(1) attenuating the activity of a polypeptide having an L-proline efflux function in the strain, wherein the polypeptide having the activity of the L-proline efflux function is:
A) a polypeptide having an amino acid sequence set forth in SEQ ID NO: 1 or SEQ ID NO: 2 and having the activity of the L-proline efflux function; or
B) a polypeptide having some amino acids added or deleted at either or both ends of the polypeptide of A) but still having the activity of the L-proline efflux function; or
C) a polypeptide having higher than 90%, 95%, 96%, 97%, 98%, or 99% homology to the polypeptide of A) and derived from *Corynebacterium* with the activity of an L-proline efflux function;
(2) relieving or attenuating feedback inhibition by L-proline on glutamate kinase; and
(3) introducing a gene encoding a proline hydroxylase into the strain obtained in step (2); preferably, the activity of glutamate kinase and/or glutamate-semialdehyde dehydrogenase and/or pyrroline-5-carboxylate reductase in the host cell is enhanced.

9. The method for constructing the strain for producing hydroxyproline as claimed in claim 8, wherein the proline hydroxylase is *trans*-proline-4-hydroxylase having a nucleotide sequence as set forth in SEQ ID NO: 5.
